Europäisches Patentamt

**European Patent Office**

**Office européen des brevets**

(19)

(11) Veröffentlichungsnummer: **0 053 314**
**B1**

(12) **EUROPÄISCHE PATENTSCHRIFT**

(45) Veröffentlichungstag der Patentschrift:
**16.05.84**

(21) Anmeldenummer: **81109689.0**

(22) Anmeldetag: **14.11.81**

(51) Int. Cl.³: **C 07 C 143/70**

(54) **Verfahren zur Chlorsulfonierung von Diphenyl und Diphenylether.**

(30) Priorität: **27.11.80 DE 3044697**

(43) Veröffentlichungstag der Anmeldung:
**09.06.82 Patentblatt 82/23**

(45) Bekanntmachung des Hinweises auf die Patenterteilung:
**16.05.84 Patentblatt 84/20**

(84) Benannte Vertragsstaaten:
**DE FR GB**

(56) Entgegenhaltungen:
**DE - A - 2 635 279**
**DE - A - 2 721 429**
**DE - A - 2 743 540**

(73) Patentinhaber: **BAYER AG, Konzernverwaltung RP
Patentabteilung, D-5090 Leverkusen 1 Bayerwerk (DE)**

(72) Erfinder: **Arend, Günter, Dr., Claudiusstrasse 42,
D-4047 Dormagen (DE)**
Erfinder: **Uhrhan, Paul, Dr., Brunnenweg 27,
D-5068 Odenthal (DE)**

Verfahren zur Chlorsulfonierung von Diphenyl und Diphenylether

Die vorliegende Erfindung betrifft ein Verfahren zur Chlorsulfonierung von Diphenyl und Diphenylether mit Chlorsulfonsäure in Gegenwart von Thionylchlorid.

Aus der DE-OS 2 721 429 ist ein Verfahren zur Herstellung von aromatischen Sulfonsäurechloriden bekannt, bei dem man die Ausgangsverbindung mit etwa der äquimolaren Menge eines Sulfonierungsmittels, bezogen auf jede einzuführende Sulfonsäuregruppe, und mit einem Überschuß an Thionylchlorid umsetzt, wobei das Sulfonierungsmittel und das Thionylchlorid vorgelegt oder gleichzeitig mit der aromatischen Verbindung eingesetzt werden.

Bei diesem Verfahren erhält man jedoch das 4,4'-Diphenyldisulfochlorid nur zu 65% der Theorie (vgl. Beispiel 15 der Tabelle 2), was für eine technische Anwendung des Verfahrens unbefriedigend ist.

Weiterhin ist aus der DE-OS 2 743 540 ein Verfahren zur Herstellung von aromatischen Sulfonsäurechloriden bekannt, demgemäß man das Ausgangsprodukt mit Chlorsulfonsäure und Thionylchlorid umsetzt, wobei man zuerst etwa stöchiometrische Mengen der aromatischen Verbindung und Chlorsulfonsäure, gegebenenfalls in Gegenwart eines Lösungsmittels und gegebenenfalls in Gegenwart eines Sulfonierungshilfsmittels, zur Reaktion bringt und dann mit überschüssigem Thionylchlorid umsetzt.

Auch bei diesem Verfahren erhält man das 4,4'-Diphenyldisulfonsäuredichlorid nur in unbefriedigenden Ausbeuten von 62% der Theorie (vgl. z. B. Beispiel 20). Nach dem Beispiel 26 der DE-OS erhält man das 4,4'-Diphenyletherdisulfonsäuredichlorid in einer Ausbeute von 97% der Theorie. Hierbei ist jedoch zu berücksichtigen, daß sich diese Ausbeute auf das Rohprodukt bezieht. Nachteilig bei dem in Beispiel 26 beschriebenen Verfahren ist jedoch, daß die Umsetzung in Gegenwart eines Lösungsmittels, nämlich Ethylenchlorid, durchgeführt werden muß. Der Zusatz eines Lösungsmittels bei der Umsetzung von Chlorsulfonsäure mit Diphenylether gemäß dem Beispiel 26 der DE-OS 2 743 540 ist aber für die angegebene hohe Ausbeute des Reaktionsproduktes entscheidend. Wie eigene Nacharbeitungen zeigen, gelingt es nicht, 4,4'-Diphenyletherdisulfonsäuredichlorid in solch hohen Ausbeuten herzustellen, wenn ohne Zusatz eines inerten organischen Lösungsmittels unter den Reaktionsbedingungen gearbeitet wird, wie sie in dem Beispiel 26 der DE-OS angegeben werden.

Ein weiteres Verfahren zur Herstellung von aromatischen Sulfonsäurechloriden ist aus der DE-OS 2 743 541 bekannt. Danach setzt man die Ausgangsverbindung mit Chlorsulfonsäure und Phosgen um, wobei man zuerst etwa stöchiometrische Mengen der aromatischen Verbindung mit Chlorsulfonsäure, gegebenenfalls in Gegenwart eines Lösungsmittels und gegebenenfalls in Gegenwart eines Sulfonierungshilfsmittels, zur Reaktion bringt und dann in Gegenwart eines Katalysators mit Phosgen umsetzt. Nachteilig bei diesem Verfahren ist das Arbeiten in Gegenwart von Phosgen, was besondere sicherheitstechnische Maßnahmen erfordert. Außerdem ist Phosgen weniger reaktiv als Thionylchlorid, so daß man bei höheren Reaktionstemperaturen arbeiten muß, was Nebenreaktionen, wie die Sulfonbildung, begünstigt.

Ein ähnliches Verfahren ist auch aus der DE-OS 2 743 542 bekannt. Gemäß dieser Offenlegungsschrift werden aromatische Sulfonsäurechloride dadurch hergestellt, daß man aromatische Sulfonsäuren mit Phosgen in Gegenwart von Katalysatoren aus der Klasse der N,N-Dialkylcarbonsäureamide in Gegenwart eines Sulfonierungsmittels umsetzt.

Auch dieses Verfahren arbeitet mit Phosgen, was, wie erwähnt, besondere sicherheitstechnische Maßnahmen erfordert und außerdem den Nachteil besitzt, daß auch hier Nebenreaktionen, wie die Sulfonbildung, in größerem Maße ablaufen als bei Verwendung von Thionylchlorid.

Es wurde nun ein Verfahren zur Herstellung von 4,4'-Diphenyldisulfonsäuredichlorid und 4,4'-Diphenyletherdisulfonsäuredichlorid durch Chlorsulfonierung von Diphenyl beziehungsweise Diphenylether mit Chlorsulfonsäure in Gegenwart von Thionylchlorid gefunden, das dadurch gekennzeichnet ist, daß man die Umsetzung mit 1,8 bis 2,1 Mol Chlorsulfonsäure in Gegenwart von 7 bis 20 Mol Thionylchlorid pro Mol Ausgangsprodukt durchführt, wobei man zunächst bei −10 bis +20°C sulfoniert, anschließend bei Rückflußtemperatur, gegebenenfalls in Anwesenheit von 0,001 bis 0,5 Mol eines N,N-Dialkylcarbonsäureamids pro Mol Sulfonierungsprodukt, chloriert und dann das Reaktionsgemisch aufarbeitet.

Bevorzugt setzt man in das erfindungsgemäße Verfahren 1,95 bis 2,05 Mol Chlorsulfonsäure und 8 bis 12 Mol Thionylchlorid pro Mol Ausgangsprodukt ein.

Die Umsetzung wird im allgemeinen so durchgeführt, daß man zunächst bei −10 bis +20°C, vorzugsweise bei 0 bis 10°C, das Diphenyl oder den Diphenylether sulfoniert und anschließend die dabei entstandene Disulfonsäure unter Anstieg der Reaktionstemperatur bis auf Rückflußtemperatur (etwa 70°C) chloriert.

Bei der Chlorierung der Disulfonsäure kann es vorteilhaft sein, wenn man in Gegenwart eines N,N-Dialkylcarbonsäureamids, wie Dimethylformamid, Dimethylacetamid, Tetramethylharnstoff und N-Methylpyrrolidon, bevorzugt Dimethylacetamid und N-Methylpyrrolidon, arbeitet.

Dabei können die N,N-Dialkylcarbonsäureamide sowohl einzeln als auch im Gemisch untereinander eingesetzt werden.

Bevorzugt setzt man N,N-Dialkylcarbonsäureamide in einer Menge von 0,01 bis 0,1 Mol N,N-Dialkylcarbonsäureamid pro Mol Disulfonsäure ein.

Das Ende der Sulfonierungsreaktion kann man an dem Aufhören der Chlorwasserstoffentwicklung erkennen. Die Sulfonierungsdauer beträgt etwa 2 bis 5 Stunden und hängt insbesondere von der gewählten Reaktionstemperatur ab.

Nach dem Ende der Sulfonierungsreaktion erhält man die entsprechenden Disulfonsäuren in kristalliner Form als Aufschlämmung in überschüssigem Thionylchlorid. Dabei ist es von besonderer Bedeutung, daß mindestens etwa 7 Mol Thionychlorid pro Mol Ausgangsprodukt im Reaktionsgemisch vorhanden sind. Andernfalls würde man keine kristalline Aufschlämmung der Disulfonsäuren erhalten, sondern eine nicht-rührbare, erstarrte Masse, die eine kontrollierte Chlorierung nicht mehr ermöglicht.

Wie zuvor erwähnt, setzt man die bei der Sulfonierung erhaltene Disulfonsäure dann mit dem im Überschuß eingesetzten Thionylchlorid weiter zum Disulfonsäuredichlorid um.

Läßt man die Temperatur dabei bis auf Rückflußtemperatur ansteigen, so ist die Chlorierung nach etwa 5 Stunden beendet; arbeitet man bei ca. 50°C, so dauert die Chlorierung etwa 15 bis 20 Stunden.

In einer bevorzugten Ausführungsform des erfindungsgemäßen Verfahrens legt man das Thionylchlorid zum größten Teil (zu etwa 60 bis 80% der einzusetzenden Menge) zusammen mit bis zu etwa 10% der einzusetzenden Chlorsulfonsäuremenge im Reaktor vor und läßt bei 0 bis 10°C gleichzeitig, aber getrennt voneinander, den Rest der Chlorsulfonsäure und eine 40gew.-%ige Lösung des Diphenyls oder des Diphenylethers im restlichen Thionylchlorid innerhalb von etwa 1 bis 3 Stunden zulaufen. Nach weiteren 1 bis 2 Stunden bei etwa 0 bis etwa 10°C ist die Sulfonierung beendet. Das Reaktionsgemisch wird nun aufgeheizt, dabei entweicht Schwefeldioxid und Chlorwasserstoff und die bei der Sulfonierung ausgefallene Disulfonsäure geht in Lösung. Die Bildung des Disulfonsäuredichlorids kann durch Zusatz der oben erwähnten N,N-Dialkylcarbonsäureamide beschleunigt werden.

Die Aufarbeitung der Disulfonsäuredichloride kann auf verschiedene Weise erfolgen.

Zur Isolierung des 4,4'-Diphenyldisulfonsäuredichlorids, das einen hohen Schmelzpunkt (Fp.: 200°C) und eine relativ geringe Löslichkeit im Thionylchlorid besitzt, kann man das Reaktionsgemisch auf Eis oder in Eiswasser gießen, wobei das 4,4'-Diphenyldisulfonsäuredichlorid in gut filtrierbarer Form anfällt.

Zur Isolierung von Diphenyletherdisulfonsäuredichlorid destilliert man vorteilhafterweise den Überschuß an Thionylchlorid ab, steigert die Sumpftemperatur bis ca. 135°C und entgast im Vakuum. Das 4,4'-Diphenyletherdisulfonsäuredichlorid fällt als Schmelze an, welche man auf Blechen erstarren läßt.

In einer anderen, bevorzugten Ausführungsform versetzt man die heiße Schmelze nach Abdestillieren des überschüssigen Thionylchlorids aus dem Reaktionsgemisch mit einem inerten, organischen Lösungsmittel, wie Methylenchlorid, Chloroform, Tetrachlorkohlenstoff, Benzol, Toluol, Xylol, Chlorbenzol, Diethylether und/oder Dibutylether, bevorzugt Methylenchlorid und/oder Chloroform, so daß man eine entsprechende Lösung des Diphenyletherdisulfonsäuredichlorids erhält. Bei geeigneter Reaktionsführung kann man das Disulfonsäuredichlorid aus dieser Lösung durch Abkühlen der Lösung auf etwa −10 bis 25°C auskristallisieren lassen. Man erhält auf diese Weise z. B. ein besonders reines 4,4'-Diphenyletherdisulfonsäuredichlorid.

Man kann die Disulfonsäuredichloride auch vorteilhafterweise so isolieren, daß man die rohe Disulfonsäuredichloridlösung in Wasser eingießt, wobei das überschüssige Thionylchlorid hydrolysiert wird und das Disulfonsäuredichlorid ausfällt. Bei dieser Verfahrensweise ist es möglich, das Thionylchlorid vorher teilweise abzudestillieren, wobei es sich als zweckmäßig erwiesen hat, eine bis auf 50% aufkonzentrierte Lösung des Disulfonsäuredichlorids in Thionylchlorid in Wasser einzurühren. Das aus dem Wasser ausfallende Disulfonsäuredichlorid wird filtriert und anschließend neutral gewaschen.

Nach dem erfindungsgemäßen Verfahren werden 4,4'-Diphenyldisulfonsäuredichlorid und 4,4'-Diphenyletherdisulfonsäuredichlorid in hoher Reinheit und guter Ausbeute erhalten.

Man kann daher die erhaltenen Disulfonsäuredichloride ohne vorhergehende Reinigung direkt durch Umsetzung mit Hydrazin in die entsprechenden Dihydrazide überführen, die als Treibmittel zur Herstellung von Schaumstoffen in der Gummi- und Kunststoffindustrie eingesetzt werden.

Die nachfolgenden Beispiele sollen das erfindungsgemäße Verfahren verdeutlichen.

Beispiel 1

In einem Reaktionsgefäß legt man 526 ml (7,2 Mol) Thionylchlorid und 9 ml Chlorsulfonsäure vor. Unter Rühren tropft man bei 0 bis 10°C gleichzeitig 127 ml (2,04 Mol) Chlorsulfonsäure und eine Lösung von 170 g (1,0 Mol) Diphenylether in 200 ml (2,8 Mol) Thionylchlorid innerhalb von 60 Minuten hinzu. Ohne Qualitätseinbuße kann man auch die Gesamtmenge Thionylchlorid vorlegen und den Diphenylether in fester Form eintragen oder als 30°C warme Schmelze zutropfen.

Die Disulfonsäure fällt als grau-grüner Niederschlag aus, die Mischung bleibt gut rührbar. Man läßt eine Stunde nachreagieren, setzt 4 ml N-Methylpyrrolidon oder Dimethylacetamid hinzu und kocht bis zur Beendigung der SO₂-Entwicklung ca. 5,5 Stunden am Rückfluß. Nun destilliert man den SOCl₂-Überschuß ab und entgast die Schmelze vollständig im Vakuum bei 125 bis 135°C.

Die Schmelze wird auf Emaille-Bleche gegossen und erstarren lassen. Das Produkt kristallisiert innerhalb von 3 bis 5 Stunden durch.

| Chlorgehalt: | 18,5% (berechnet 19,3%) |
| Ausbeute: | 360 g ≙ 99,7% |
| | (≙ 95,5% korrigiert auf CI-Gehalt) |
| Fp.: | 120 bis 123° C |
| Reinheit: | 95,5% |

### Beispiel 2

Analog zu Beispiel 1 setzt man miteinander um:

| 726 ml | (10,0 Mol SOCl$_2$, |
| 127 ml | (1,0 Mol) Chlorsulfonsäure und |
| 170 g | (1,0 Mol) Diphenylether |

In die entgaste Schmelze von ca. 130° C läßt man unter Verdampfungskühlung 100 ml Methylenchlorid langsam zulaufen. Die Temperatur fällt rasch ab, dabei kristallisiert das Sulfochlorid aus. Man kühlt unter Rühren weiter auf Raumtemperatur ab und isoliert das Produkt durch Absaugen.

| Ausbeute: | 237 g ≙ 70% weiße Kristalle (Reinheit: 99,5%) |
| FP.: | 133° C |
| CI-Gehalt: | 19,2% (berechnet 19,3%) |

Beim Einengen der Mutterlauge auf ein Drittel des Ausgangsvolumens oder nach Versetzen der Mutterlauge mit dem gleichen Volumenteil Ligroin fallen nochmals 33 g (≙ 9%), Fp.: 123 bis 126° C, Disulfochlorid an.

Versetzt man die Schmelze mit 800 ml Methylenchlorid, so erhält man eine ca. 25%ige Lösung des Disulfochlorids. Beim Einengen einer Probe enthält man einen Rückstand mit einem Schmelzbereich von 118 bis 127° C, (CI-Gehalt: 19,1% entsprechend einer Reinheit von 99,0%).

### Beispiel 3

Beispiel 2 wird ohne Zusatz von N-Methylpyrrolidon bzw. Dimethylacetamid wiederholt. Die Lösung des Dichlorids im überschüssigen Thionylchlorid wird auf 25° C abgekühlt und in 3 l Wasser bei 0 bis 5° C eingetropft. Die entstehende beige Kristallmasse wird abgesaugt und mit kaltem Wasser neutral gegen Kongorot gewaschen.

| Ausbeute: | 500 g feuchtes Produkt, das 360 g trockenem Produkt entspricht (≙ 98% der Theorie), |
| Fp.: | 120 bis 125° C |
| verseifbares Chlor: | 91% (≙ Reinheit von 91%) |

### Beispiel 4

In einem Reaktionsgefäß legt man 263 ml Thionylchlorid und 4,5 ml Chlorsulfonsäure vor. Bei 0 bis 10° C tropft man innerhalb von 1 Stunde gleichzeitig 63,5 ml Chlorsulfonsäure und eine Lösung von 77 g Diphenyl in 100 ml Thionylchlorid hinzu. Man läßt 1 Stunde bei 0 bis 10° C nachreagieren und erhält eine hellgraue bis grünliche Suspension der ausgefallenen Diphenyldisulfonsäure.

Man setzt 2 ml N-Methylpyrrolidon oder Dimethylacetamid zu und heizt innerhalb von 4 Stunden auf 65° C. Unter SO$_2$- und HCl-Entwicklung löst sich der Niederschlag auf. Zur Vervollständigung läßt man noch 4 Stunden bei 65° C reagieren und kühlt dann auf 20 bis 30° C ab. Die dunkelgrüne Lösung wird unter Rühren bei 0 bis 5° C auf Eiswasser getropft; dabei fällt das Disulfochlorid als hellockerfarbener Feststoff aus.

| Ausbeute: | 279 g feuchtes Produkt, das 167 g trocknem Produkt entspricht (≙ 95% der Theorie) |
| Fp.: | 174 bis 185° C |
| Chlorgehalt: | 19,6% im Trockengut (berechnet: 20,2%) (≙ Reinheit von ca. 90%) |

## Patentansprüche

1. Verfahren zur Herstellung von 4,4'-Diphenyletherdisulfonsäuredichlorid und 4,4C'-Diphenyletherdisulfonsäuredichlorid durch Chlorsulfonierung von Diphenyl beziehungsweise Diphenylether mit Chlorsulfonsäure in Gegenwart von Thionylchlorid, dadurch gekennzeichnet, daß man die Umsetzung mit 1,8 bis 2,1 Mol Chlorsulfonsäure in Gegenwart von 7 bis 20 Mol Thionylchlorid pro Mol Ausgangsprodukt durchführt, wobei man zunächst bei −10 bis +20° C sulfoniert, anschließend bei Rückflußtemperatur, gegebenenfalls in Anwesenheit von 0,001 bis 0,5 Mol eines N,N-Dialkylcarbonsäureamids pro Mol Sulfonierungsprodukt, chloriert und dann das Reaktionsgemisch aufarbeitet.

2. Verfahren nach Anspruch 1, dadurch gekennzeichnet, daß man die Umsetzung mit 1,95 bis 2,05 Mol Chlorsulfonsäure pro Mol Ausgangsprodukt durchführt.

3. Verfahren nach Ansprüchen 1 und 2, dadurch gekennzeichnet, daß man die Umsetzung in Gegenwart von 8 bis 12 Mol Thionylchlorid pro Mol Ausgangsprodukt durchführt.

4. Verfahren nach Ansprüchen 1 bis 3, dadurch gekennzeichnet, daß man die Sulfonierung bei 0 bis 10° C durchführt.

5. Verfahren nach Ansprüchen 1 bis 4, dadurch gekennzeichnet, daß man als N,N-Dialkylcarbonsäureamid, Dimethylformamid, Dimethylacetamid, Tetramethylharnstoff und/oder N-Methylpyrrolidon einsetzt.

6. Verfahren nach Ansprüchen 1 bis 5, dadurch gekennzeichnet, daß man pro Mol Sulfonierungsprodukt 0,01 bis 0,1 Mol N,N-Dialkylcar- bonsäureamid einsetzt.

7. Verfahren nach Ansprüchen 1 bis 6, dadurch gekennzeichnet, daß man 60 bis 80% des einzusetzenden Thionylchlorids zusammen mit bis zu 10% der einzusetzenden Chlorsulfonsäuremenge im Reaktor vorlegt und bei 0 bis 10°C gleichzeitig, aber getrennt voneinander, den Rest der Chlorsulfonsäure und eine 40gew.-%ige Lösung des Diphenyls oder des Diphenylethers in restlichem Thionylchlorid zulaufen läßt.

8. Verfahren nach Ansprüchen 1 bis 7, dadurch gekennzeichnet, daß man das Reaktionsgemisch in Eiswasser einrührt, das ausfallende Disulfonsäuredichlorid abfiltriert und mit Wasser wäscht.

9. Verfahren nach Ansprüchen 1 bis 8, dadurch gekennzeichnet, daß man aus dem Reaktionsgemisch das überschüssige Thionylchlorid abdestilliert, die zurückbleibende Schmelze mit einem inerten organischen Lösungsmittel versetzt und das Disulfonsäuredichlorid durch Abkühlen der Lösung auf −10 bis +25°C isoliert.

## Claims

1. Process for the preparation of 4,4'-diphenyldisulphonic acid dichloride and 4,4'-diphenyl ether disulphonic acid dichloride by the chlorosulphonation of diphenyl or, respectively, diphenyl ether with chlorosulphonic acid in the presence of thionyl chloride, characterized in that the reaction is carried out with 1.8 to 2.1 mols of chlorosulphonic acid in the presence of 7 to 20 mols of thionyl chloride per mol of starting material, sulphonation at −10 to +20°C being carried out first, followed by chlorination at the reflux temperature, if necessary in the presence of 0.001 to 0.5 mol of a N,N-dialkylcarboxylic acid amide per mol of sulphonation product, and the reaction mixture then being worked up.

2. Process according to claim 1, characterized in that the reaction is carried out using 1.95 to 2.05 mols of chlorosulphonic acid per mol of starting material.

3. Process according to claims 1 and 2, characterized in that the reaction is carried out in the presence of 8 to 12 mols of thionyl chloride per mol of starting material.

4. Process according to claims 1 to 3, characterized in that the sulphonation is carried out at 0 to 10°C.

5. Process according to claims 1 to 4, characterized in that dimethylformamide, dimethylacetamide, tetramethylurea and/or Nmethylpyrrolidone are employed as the N,N-dialkylcarboxylic acid amide.

6. Process according to claims 1 to 5, characterized in that 0.01 to 0.01 mol of N,N-dialkylcarboxylic aced amide is employed per mol of sulphonation product.

7. Process according to claims 1 to 6, characterized in that 60 to 80% of the thionyl chloride to be used is introduced into the reaction vessel with up to 10% of the quantity of chlorosulphonic acid to be used and the remainder of the chlorosulphonic acid and a 40% strength by weight solution of the diphenyl or the diphenyl ether in the remaining thionyl chloride are allowed to run simultaneously, but separately from each other, into the solution at 0 to 10°C.

8. Process according to claims 1 to 7, characterised in that the reaction mixture is stirred into ice water and the disulphonic acid dichloride precipitated is filtered off and washed with water.

9. Process according to claims 1 to 8, characterized in that the excess thionyl chloride is distilled off from the reaction mixture, an inert organic solvent is added to the melt which remains and the disulphonic acid dichloride is isolated by cooling the solution to −10 to +25°C.

## Revendications

1. Procédé pour la fabrication de dichlorure d'acide 4,4'diphényldisulfonique et de chlorure d'acide 4,4'-oxydiphényldisulfonique par chlorosulfonation du diphényle ou de l'oxyde de diphényle par l'acide chlorosulfonique en présence de chlorure de thionyle, caractérisé en ce que l'on met en oeuvre la réaction avec 1,8 à 2,1 moles d'acide chlorosulfonique en présence de 7 à 20 moles de chlorure de thionyle par mole du produit de départ, en sulfonant d'abord à une température de − 10 à +20°C, en chlorurant ensuite à la température de reflux, éventuellement en présence de 0,001 à 0,5 mole d'un N,N-dialkyl-carboxamide par mole de produit de sulfonation, et en traitant ensuite le mélange de réaction.

2. Procédé selon la revendication 1, caractérisé en ce que l'on met en oeuvre la réaction avec 1,95 à 2,05 moles d'acide chlorosulfonique par mole de produit de départ.

3. Procédé selon les revendications 1 et 2, caractérisé en ce que l'on met en oeuvre la réaction en présence de 8 à 12 moles de chlorure de thionyle par mole de produit de départ.

4. Procédé selon les revendications 1 à 3, caractérisé en ce que l'on met en oeuvre la sulfonation à une température de 0 à 10°C.

5. Procédé selon les revendications 1 à 4, caractérisé en ce que l'on utilise comme N,N-dialkylcarboxamide le diméthylformamide, le diméthylacétamide, la tétraméthylurée et/ou la N-méthylpyrrolidone.

6. Procédé selon les revendications 1 à 5, caractérisé en ce que l'on utilise 0,01 à 0,1 mole de N,N-dialkylcarboxamide par mole de produit de sulfonation.

7. Procédé selon les revendications 1 à 6, caractérisé en ce que l'on charge dans le réacteur 60 à 80% du chlorure de thionyle à utiliser avec jusqu'à 10% de la quantité d'acide chlorosulfonique à utiliser et on fait couler simultanément, mais séparément l'un de l'autre, le reste de l'acide chlorosulfonique et une solution à 40% en poids de diphényle ou de l'oxyde de diphényle dans le reste du chlorure de thionyle à 0−10°C.

8. Procédé selon les revendications 1 à 7, caractérisé en ce que l'on verse le mélange de réaction dans l'eau glacée en agitant, on sépare par filtration le dichlorure d'acide disulfonique qui précipite et on le lave à l'eau.

9. Procédé selon les revendications 1 à 8, caractérisé en ce que l'on chasse l'excès de chlorure de thionyle du mélange de réaction par distillation, on ajoute à la masse fondue résiduelle un solvant organique inerte et on isole le dichlorure d'acide disulfonique par refroidissement de la solution à une température de — 10 à + 25° C.